# FASCICULE DE BREVET EUROPEEN

(11) **EP 3 994 442 B1**
(45) Date de publication et mention de la délivrance du brevet: **09.08.2023**
(21) Numéro de dépôt: 20739282.0
(22) Date de dépôt: 30.06.2020
(51) Int. Cl.: G01N 1/44, G01N 33/24, G01N 1/04, G01N 1/02

(54) **DISPOSITIF DE PRODUCTION DE CO2 GAZEUX À PARTIR DE CARBONATES POUR ANALYSE ISOTOPIQUE (DELTA13C ET DELTA18O) SUR SITE ET PROCÉDÉ ASSOCIÉ**
VORRICHTUNG ZUR HERSTELLUNG VON CO2-GAS AUS KARBONATEN FÜR IN-SITU ISOTOPISCH ANALYSE (DELTA13C UND DELTA18O) UND VERFAHREN DAVON
DEVICE FOR PRODUCTION OF CO2 GAS FROM CARBONATES FOR IN-SITU ISOTOPIC ANAYLSIS (DELTA13C AND DELTA18O) AND METHOD THEREOF

(30) Priorité: 01.07.2019 FR 1907289
(43) Date de publication de la demande: 11.05.2022
(73) Titulaire: Université de Bourgogne, 21000 Dijon (FR); Centre national de la recherche scientifique, 75016 Paris (FR); Université de Bretagne Occidentale, 29238 Brest (FR)
(72) Inventeur: MUSSET, Olivier, 21000 DIJON (FR); THOMAZO, Christophe, 21000 DIJON (FR); SANS JOFRE, Pierre, 94220 CHARENTON-LE-PONT (FR); COCQUEREZ, Théophile, 21110 LONGECOURT-EN-PLAINE (FR); LALONDE, Stefan, 29200 BREST (FR)
(74) Mandataire: IPAZ
(86) Numéro de dépôt international: PCT/EP2020/068346
(87) Numéro de publication internationale: WO 2021/001344

(56) Documents cités:
- WO-A1-2015/189283
- WO-A1-2018/082136
- CN-A- 104 458 979
- US-A- 5 656 186
- US-A1- 2002 163 735
- US-A1- 2012 206 722
- US-B1- 10 222 337
- US-B2- 8 642 918

## Description

### Domaine technique

La présente invention se rapporte à la préparation d'échantillons gazeux à partir d'échantillons solides, notamment à la préparation d'échantillons gazeux destinés à être examinés par analyse isotopique, par exemple par spectrométrie de masse ou spectroscopie optique.

La présente invention vise, en particulier, la préparation d'échantillons gazeux à partir d'échantillons solides, principalement des minéraux contenant du carbone et/ou de l'oxygène.

Plus particulièrement, la présente invention concerne la préparation d'échantillons gazeux à partir d'échantillons rocheux contenant des carbonates destinés à être examinés par analyse isotopique du carbone et/ou de l'oxygène.

### État de la technique antérieure

On connait dans l'état de la technique antérieure la préparation d'échantillons gazeux pour l'analyse isotopique d'échantillons solides, en particulier d'échantillons minéraux, par traitement chimique. Un fragment broyé de l'échantillon à analyser est traité chimiquement par dissolution dans un acide, typiquement de l'acide ortho-phosphorique, ou par chauffage. Cette réaction produit du dioxyde de carbone qui est ensuite injecté dans un spectromètre de masse pour analyse isotopique du carbone et/ou de l'oxygène.

Un inconvénient de cette technique réside dans le fait qu'il est nécessaire de prélever sur le terrain un fragment de l'échantillon à analyser puis de l'envoyer en laboratoire afin qu'il soit broyé et préparé, en particulier via des traitements visant à retirer la matière organique, avant d'être traité chimiquement. Ces étapes introduisent une à plusieurs semaines de délai entre le prélèvement et les résultats, rendant l'analyse chronophage et coûteuse en termes de consommables et de ressources humaines.

Un autre inconvénient de cette technique est qu'elle est échantillon dépendante. Elle nécessite l'intervention d'un expert en géologie pour élaborer le protocole d'analyse et le choix de l'acide en fonction de l'appréciation qu'il aura faite quant à la nature de la roche. Il est courant d'effectuer plusieurs traitements compte tenu de l'incertitude liée à la nature de la roche.

On connaît également dans l'état de la technique la préparation d'échantillons gazeux pour l'analyse isotopique d'échantillons solides, en particulier d'échantillons minéraux, par ablation laser. La technique la plus répandue pour réaliser ce type d'analyse est la spectrométrie de masse à plasma à couplage inductif. Cette technique est mise en oeuvre dans un dispositif comprenant un laser d'ablation couplé à un spectromètre de masse. Les lasers utilisés dans ce type de dispositif sont des lasers solides déclenchés principalement Nd:YAG. Un exemple peut être trouvé dans US 2002/163735 A1 concernant l'application des lasers en chimie analytique, et plus particulièrement le mécanisme d'ablation laser directe du solide au gaz induit optiquement.

Comme pour le traitement chimique, un inconvénient de l'ablation laser réside dans le fait qu'il est nécessaire de prélever sur le terrain un fragment de l'échantillon à analyser et de l'envoyer en laboratoire afin qu'il soit préparé pour analyse, ces techniques utilisent des appareils dont l'encombrement et le poids rendent impossible tout déplacement sur site.

Un inconvénient supplémentaire inhérent aux techniques de préparation d'échantillons gazeux pour l'analyse isotopique par ablation laser est lié au phénomène de fractionnement isotopique des éléments. Ce fractionnement est dû aux recombinaisons et aux variations du taux de récupération qui entraine une modification du ratio isotopique dans l'échantillon gazeux par rapport au ratio isotopique réel de l'échantillon solide. Cela entraîne des résultats d'analyse isotopique biaisés.

Un autre inconvénient des techniques de préparation d'échantillons gazeux de l'état de l'art tient au fait que l'efficacité de la réaction d'extraction des isotopes pour certains lasers n'est pas totale. Ce manque d'efficacité de l'extraction est pénalisant car celui-ci peut induire un fractionnement isotopique et fausser les analyses.

Un autre inconvénient des techniques de préparation d'échantillons gazeux de l'état de l'art tient au fait qu'elles sont matrices dépendantes. La préparation n'est pas identique pour tout échantillon solide mais varie d'un type d'échantillon à l'autre en raison des effets de matrice. Cet inconvénient nécessite l'utilisation de facteurs correctifs lors de l'analyse isotopique d'un élément.

Un autre inconvénient des techniques de préparation d'échantillons gazeux de l'état de l'art réside dans le fait qu'une étape de chromatographie en phase gazeuse est réalisée sur l'échantillon gazeux préalablement à l'analyse isotopique.

Un autre inconvénient des techniques de préparation d'échantillons gazeux de l'état de l'art est qu'elles sont sensibles à la pollution organique présente à la surface ou dans les échantillons solides. Les techniques de l'état de l'art nécessitent donc de préparer la surface de l'échantillon avant de procéder à la préparation de l'échantillon gazeux.

Un but de l'invention est notamment de :
- pallier à au moins un des inconvénients des techniques de préparation d'échantillons gazeux de l'état de l'art, et/ou
- de proposer une préparation d'échantillons gazeux insensible aux effets de matrice, et/ou
- de proposer une préparation d'échantillons gazeux ne nécessitant pas de préparation, c'est-à-dire d'étape de nettoyage visant à retirer la pollution organique de surface, et/ou
- de proposer une préparation d'échantillons gazeux insensible à la présence de carbone organique, et/ou
- de proposer une préparation d'échantillons gazeux dont l'efficacité de réaction est de 100%, et/ou
- de proposer un dispositif pour la préparation d'échantillons gazeux transportable sur site, permettant de l'analyse *in situ,* et/ou
- de proposer une préparation d'échantillons gazeux qui permette d'analyser directement l'échantillon gazeux sans recourir à une étape de chromatographie en phase gazeuse avant l'analyse isotopique, et/ou
- de proposer une préparation d'échantillons gazeux qui n'engendre pas de fractionnement isotopique, ou qui *a minima* engendre un fractionnement isotopique négligeable, et/ou
- de permettre une préparation d'échantillons gazeux et/ou une analyse isotopique directement sur site, par exemple sur des échantillons solides hors d'atteinte, au niveau d'une tête de forage, ou par exemple sur des échantillons solides situés dans des zones reculées, et/ou
- de permettre une analyse isotopique ne nécessitant pas l'utilisation de facteurs correctifs lors de la détermination de l'isotopie d'un élément, et en particulier du carbone.

### Présentation de l'invention

A cet effet, il est proposé un dispositif pour la préparation d'un échantillon gazeux obtenu à partir d'un échantillon solide, ledit dispositif comprenant :
- une source laser agencée pour émettre un faisceau laser apte à générer une calcination et/ou une combustion d'une partie de l'échantillon solide et inapte à engendrer une ablation laser,
- un moyen agencé pour collecter un échantillon gazeux d'un gaz émis lors de la calcination et/ou de la combustion de la partie de l'échantillon solide ;

le dispositif étant caractérisé en ce que le faisceau laser présente une irradiance inférieure à 5 MW/cm² au niveau d'une zone d'impact donnée sur une surface de l'échantillon,
et en ce qu'il comprend une fibre optique pour la propagation du faisceau laser entre la source laser et la zone d'impact donnée sur la surface de l'échantillon.

Dans la présente demande, lorsque le terme « dispositif » est utilisé seul, il désigne le dispositif pour la préparation d'échantillon gazeux selon l'invention. Selon l'invention, l'échantillon gazeux est destiné à être examiné par analyse isotopique.

Le faisceau laser en sortie de la source laser peut présenter une puissance inférieure à 500W, de préférence une puissance inférieure à 100 W. Le faisceau laser en sortie de la source laser peut présenter une puissance supérieure à 1 W.

De préférence, le faisceau laser présente, au niveau de la zone d'impact donnée sur la surface de l'échantillon, une irradiance inférieure à 1 MW/cm², de préférence encore inférieure à 500 kW/cm², de manière d'avantage préférée inférieure à 100 kW/cm², de manière encore d'avantage préférée une irradiance inférieure à 50 kW/cm², de manière d'avantage préférée entre toutes une irradiance inférieure à 20 kW/cm².

La fibre optique permet que la propagation du faisceau laser entre la source laser et la zone d'impact s'effectue de manière confinée de sorte à :
- atteindre un échantillon situé loin de la source laser, et/ou
- atteindre un échantillon hors de portée d'un utilisateur, et/ou
- adapter, en temps réel, le trajet et la zone d'impact du faisceau laser indépendamment de l'environnement.

Selon l'invention, le faisceau laser :
- permet de chauffer, au niveau de la zone d'impact, l'échantillon solide à une température supérieure à 600 °C et/ou à une température inférieure à 1100 °C,
- ne permet pas d'extraire de la matière de l'échantillon solide par ablation laser.

Il peut être entendu par « un faisceau laser apte à générer une calcination et/ou une combustion d'une partie de l'échantillon solide », un faisceau laser apte à générer principalement ou majoritairement une calcination et/ou une combustion d'une partie de l'échantillon solide.

Il est entendu par ablation laser l'extraction de matière de l'échantillon solide directement provoqué par l'interaction entre le laser et l'échantillon solide. Cette extraction est provoquée directement par le laser par exemple par formation d'un plasma laser, par pulvérisation, par évaporation ou par évaporation explosive ou par effet photochimique de type photoablatif ou par choc mécanique.

L'homme du métier saura comprendre qu'il est entendu par évaporation, générée par l'ablation laser, le passage de matière de l'échantillon solide de l'état solide à l'état gazeux (sublimation).

Selon l'invention, la source laser peut être agencée pour émettre en mode :
- continu ou quasi-continu, et/ou
- impulsionnel relaxé, et/ou
- haché.

La source laser, fonctionnant par exemple en mode impulsionnel relaxé et/ou haché, peut être agencée pour émettre pendant une durée supérieure à 10 microseconde (µs), de préférence à 50 µs, de manière d'avantage préférée à 100 µs, de manière encore d'avantage préférée à 500 µs et de manière préférée entre toutes à 1 seconde.

De préférence, la source laser est agencée pour émettre en mode continu ou quasi-continu.

Selon l'invention, la source laser est inapte à émettre en mode déclenché.

Selon l'invention, le faisceau laser, au niveau de la zone d'impact, n'est pas apte à engendrer une ablation laser, c'est-à-dire qu'il n'est pas apte à modifier la structure de l'échantillon solide et/ou à extraire de la matière de l'échantillon solide autrement que par la réaction de calcination et/ou la combustion qu'il induit au niveau de la zone d'impact donnée sur la surface de l'échantillon solide. Le faisceau peut être agencé de sorte à présenter une irradiance telle qu'elle provoque un chauffage de la zone d'impact donnée sur la surface de l'échantillon solide à une température voisine ou supérieure à 900 °C.

Selon l'invention, un rendement optique/électrique de la source laser peut être supérieur à 30 %.

De préférence, le rendement optique/électrique de la source laser est supérieur à 40 %.

Selon l'invention, la source laser peut fonctionner à basse tension et à fort courant. Il est entendu par basse tension, une valeur de tension inférieure à 50 volts et supérieure ou égale à 1 V. Il est entendu par fort courant une valeur de courant supérieure ou égale à 1 ampère et inférieure à 50 A.

La source laser peut comprendre une diode laser.

Selon l'invention :
- la source laser peut être une diode laser, ou
- la source laser peut être une fibre dopée à pompage optique.

Le faisceau laser émis par la diode laser peut être injecté dans la fibre optique pour la propagation du faisceau laser entre la source laser et la zone d'impact donnée sur la surface de l'échantillon.

La source laser peut être agencée pour émettre un faisceau laser de longueur d'onde supérieure à 0,3 µm et/ou inférieure à 5 µm.

Selon l'invention, un diamètre d'un coeur de la fibre optique peut présenter une valeur supérieure à 2 µm et/ou inférieure à 800 µm.

De préférence, le diamètre du coeur de la fibre peut être supérieur à 5 µm.

De préférence, le diamètre du coeur de la fibre peut être compris entre 50 µm et 800 µm.

Selon l'invention, une ouverture numérique de la fibre optique peut être supérieure à 0,1 et/ou inférieure à 0,5.

L'ouverture numérique de la fibre optique peut être, typiquement, de 0,22.

Le dispositif selon l'invention peut comprendre des moyens de mise en forme et de focalisation du faisceau laser étant agencés de sorte que le dispositif présente une distance focale supérieure à 5 mm.

De préférence, la distance focale du dispositif est supérieure à 5 mm et/ou inférieure à 200 mm.

De préférence encore, la distance focale du dispositif est supérieure à 10 mm.

La fibre optique et les moyens de mise en forme et de focalisation peuvent être agencés de sorte que le faisceau laser présente une longueur de Rayleigh supérieure à 0,5 mm et/ou inférieure à 10 mm.

De préférence, le faisceau laser présente une longueur de Rayleigh supérieure à 1,5 mm et/ou inférieure à 8 mm.

Le dispositif peut être agencé de sorte qu'une variation d'une distance entre l'échantillon solide et une sortie des moyens de mise en forme et de focalisation du faisceau laser inférieure à 2 mm engendre une variation d'un waist du faisceau laser inférieur à 10 %.

Le dispositif selon l'invention peut comprendre un moyen de mise en mouvement agencé pour mettre en mouvement le faisceau laser et/ou la zone d'impact l'un relativement à l'autre.

Le faisceau optique peut présenter un facteur M² supérieur à 2 et/ou inférieur à 200.

Le facteur M² renseigne sur la qualité du faisceau optique.

De préférence, le faisceau optique présente un facteur M² supérieur à 5 et/ou inférieur à 150. De préférence encore, le faisceau optique présente un facteur M² supérieur à 20 et/ou inférieur à 150.

Le dispositif peut comprendre :
- une source de lumière agencée pour illuminer au moins la zone d'impact,
- un capteur optique agencé pour imager la zone d'impact illuminée et/ou pour mesurer un ou des paramètres optiques d'une lumière réfléchie par la zone d'impact illuminée de l'échantillon solide.

La source de lumière peut être agencée pour émettre dans le visible. De préférence, la source de lumière est agencée pour émettre une lumière poly-chromatique. De préférence encore, la source de lumière est agencée pour émettre une lumière blanche.

La source de lumière agencée pour illuminer au moins la zone d'impact peut être le faisceau laser émis par la source laser.

Le faisceau laser émis par la source laser peut être utilisé comme source de lumière agencée pour illuminer au moins la zone d'impact, le faisceau laser est dans ce cas inapte à générer une calcination et/ou une combustion de l'échantillon solide. De préférence, lorsque le faisceau laser émis par la source laser est utilisé comme source de lumière agencée pour illuminer au moins la zone d'impact, le faisceau laser présente une irradiance, au niveau de la zone d'impact, inférieure à 10 W/cm². De préférence, lorsque le faisceau laser émis par la source laser est utilisé comme source de lumière agencée pour illuminer au moins la zone d'impact, le faisceau laser en sortie de la source laser présente une puissance inférieure à 1 W, de préférence une puissance de quelques centaines de milliwatts.

A titre d'exemple non limitatif, le ou les paramètres optiques de la lumière réfléchie par la zone d'impact de l'échantillon solide peuvent être un coefficient de diffusion et/ou un coefficient de réflexion et/ou un coefficient d'absorption de la zone d'impact de l'échantillon solide.

Le dispositif peut comprendre une unité de traitement agencée et/ou configurée et/ou programmée pour déterminer, à partir du ou des paramètres optiques mesurés par le capteur optique, un flux lumineux à appliquer à la zone d'impact.

Le dispositif peut comprendre un moyen agencé pour déposer de la matière sur la zone d'impact de l'échantillon solide, ladite matière étant agencée pour amorcer la calcination et/ou la combustion de la partie de l'échantillon solide contiguë à la matière déposée.

Le dispositif peut comprendre une enceinte agencée pour :
- coopérer avec le moyen agencé pour collecter l'échantillon gazeux,
- que l'échantillon solide y soit disposé à l'intérieur, ou
- être mise en contact direct avec la surface de l'échantillon solide. L'enceinte peut être hermétique ou non.

L'enceinte et/ou le moyen agencé pour collecter le gaz peuvent comprendre une ouverture.

L'enceinte et/ou le moyen agencé pour collecter le gaz peuvent être connectés à une pompe à vide de sorte à générer une dépression respectivement dans l'enceinte et/ou dans le moyen agencé pour collecter le gaz. La dépression qui peut être générée dans le moyen agencé pour collecter le gaz peut générer une aspiration au niveau de l'ouverture du moyen agencé pour collecter le gaz. La dépression qui peut être générée dans l'enceinte et/ou l'aspiration générée à l'ouverture du moyen agencé pour collecter le gaz permet de collecter le gaz émis lors de la calcination et/ou de la combustion. L'enceinte et/ou le moyen agencé pour collecter le gaz peuvent comprendre un moyen d'étanchéité agencé le long de leur ouverture respective. L'enceinte et/ou le moyen agencé pour collecter le gaz peuvent être hermétiques.

Un vide primaire ou partiel peut être généré, par la pompe à vide connectée à l'enceinte et/ou au moyen agencé pour collecter le gaz, dans l'enceinte et/ou dans le moyen agencé pour collecter le gaz.

Le dispositif peut comprendre une arrivée de gaz agencée de sorte à créer une atmosphère inerte dans l'enceinte et/ou dans le moyen agencé pour collecter le gaz et/ou au niveau de la zone d'impact de l'échantillon solide. Le gaz injecté par l'arrivée de gaz peut être un gaz inerte tel que, à titre d'exemple non limitatif, de l'azote ou un gaz rare, tel que l'hélium ou l'argon.

Selon une première variante, l'enceinte peut comprendre une pièce amovible agencée pour fermer l'ouverture. La pièce amovible peut être agencée pour fermer l'ouverture de l'enceinte dans laquelle est disposé l'échantillon solide. La pièce amovible peut être agencée pour fermer hermétiquement l'ouverture.

Selon une deuxième variante, l'ouverture du moyen agencé pour collecter le gaz peut être agencée pour être portée au contact de la surface de l'échantillon solide de sorte à fermer le moyen agencé pour collecter le gaz. L'ouverture du moyen agencé pour collecter le gaz peut être agencée pour être portée au contact de la surface de l'échantillon de sorte à fermer l'enceinte de manière hermétique.

La calcination est une réaction ne nécessitant pas d'oxygène. Aussi, selon l'invention, lorsque le dispositif est agencé de sorte que l'enceinte et/ou le moyen agencé pour collecter le gaz et/ou la zone d'impact de l'échantillon solide soit en dépression ou sous vide ou sous atmosphère inerte, le faisceau laser provoque uniquement la calcination de l'échantillon solide. Dans ce cas, la préparation de l'échantillon gazeux est insensible à la présence de pollution organique dans ou à la surface de l'échantillon

Le dispositif peut comprendre un élément optique situé sur un trajet du faisceau laser en sortie des moyens de mise en forme et de focalisation du faisceau laser, ledit élément optique étant agencé pour protéger lesdits moyens de mise en forme et de focalisation du faisceau laser.

L'élément optique peut être une lame optique. La lame optique peut comprendre un traitement antireflet, à la longueur d'onde du faisceau laser, sur chacune de ses deux faces.

L'élément optique peut être amovible de sorte à être remplacé. L'élément optique a pour fonction de protéger les moyens de mise en forme et de focalisation de la contamination engendrée par des poussières ambiantes et/ou des vapeurs produites lors de calcination et/ou de la combustion.

Selon l'invention, il est également proposé une utilisation du dispositif pour la préparation d'un échantillon gazeux selon l'invention, pour l'analyse de compositions isotopiques en carbone et/ou en oxygène contenus dans des roches carbonatées.

Selon l'invention, il est également proposé un dispositif portable pour l'analyse de compositions isotopiques en carbone et/ou en oxygène contenus dans des roches carbonatées, ledit dispositif pour l'analyse de compositions isotopiques comprenant :
- le dispositif pour la préparation d'échantillon gazeux selon l'invention,
- un spectromètre optique ou un spectromètre de masse agencé pour réaliser des analyses isotopiques d'éléments contenus dans des échantillons gazeux préparés par ledit dispositif pour la préparation d'échantillon gazeux.

Le spectromètre optique agencé pour réaliser des analyses isotopiques peut être un spectromètre optique à cavité optique (de type Cavity Ring Down Spectroscope (CRDS)). Le spectromètre optique permet, en particulier, de réaliser des analyses sur site.

Le spectromètre de masse agencé pour réaliser des analyses isotopiques peut être un spectromètre de masse à rapport isotopique (de type Isotope-Ratio Mass Spectrometer (IRMS)).

Selon l'invention, il est également proposé un procédé de préparation d'un échantillon gazeux obtenu à partir d'un échantillon solide, ledit procédé comprenant :
- une émission, par une source laser, d'un faisceau laser apte à générer une calcination et/ou une combustion d'une partie de l'échantillon solide,
- une collecte, par un moyen de collecte, d'un échantillon gazeux d'un gaz émis lors de la calcination et/ou de la combustion de la partie de l'échantillon solide ;

le procédé étant caractérisé en ce qu'une irradiance du faisceau laser émis est inférieure à 5 MW/cm² au niveau d'une zone d'impact donnée sur une surface de l'échantillon,
et en ce qu'il comprend une propagation, par une fibre optique, du faisceau laser entre la source laser et la zone d'impact donnée sur la surface de l'échantillon.

Selon l'invention, il est également proposé un procédé d'analyse isotopique comprenant :
- le procédé de préparation d'un échantillon gazeux obtenu à partir d'un échantillon solide selon l'invention,
- une analyse isotopique, par un spectromètre optique ou un spectromètre de masse, d'éléments contenus dans des échantillons gazeux préparés par le dispositif pour la préparation d'échantillon gazeux.

### Description des figures

D'autres avantages et particularités de l'invention apparaîtront à la lecture de la description détaillée de mises en oeuvre et de modes de réalisation nullement limitatifs, et des dessins annexés suivants :
[Fig. 1] la Figure 1 illustre un dispositif pour la préparation d'un échantillon gazeux selon l'invention et d'un dispositif d'analyse isotopique selon l'invention,
[Fig. 2] la Figure 2 illustre une vue de biais de la partie du dispositif pour la préparation d'un échantillon gazeux selon l'invention situé en sortie de la fibre optique du dispositif d'analyse isotopique,
[Fig. 3] la Figure 3 illustre une vue de côté de la partie du dispositif pour la préparation d'un échantillon gazeux selon l'invention situé en sortie de la fibre optique du dispositif d'analyse isotopique,
[Fig. 4] la Figure 4 illustre une vue de biais d'une enceinte destinée à être utilisée en coopération avec le dispositif pour la préparation d'un échantillon gazeux selon l'invention,
[Fig. 5] la Figure 5 est un graphique représentant l'ajustement réalisé entre les ratios ¹³C/¹²C mesurés sur des échantillons gazeux préparés par un appareil commercial « KIEL IV Carbonate Device » à partir de roches d'un lot de roches, dit panel, et les ratios ¹³C/¹²C mesurés sur des échantillons gazeux préparés par le dispositif pour la préparation d'échantillons gazeux 1 selon l'invention à partir des roches du panel,
[Fig. 6] la Figure 6 est un graphique représentant l'ajustement réalisé entre les ratios ¹⁸O/¹⁶O mesurés sur des échantillons gazeux préparés par l'appareil commercial « KIEL IV Carbonate Device » à partir de roches du panel et les ratios ¹⁸O/¹⁶O mesurés sur les échantillons gazeux préparés par le dispositif pour la préparation d'échantillons gazeux 1 selon l'invention à partir des roches du panel.

### Description des modes de réalisation

Les modes de réalisation décrits ci-après étant nullement limitatifs, on pourra notamment considérer des variantes de l'invention ne comprenant qu'une sélection de caractéristiques décrites, isolées des autres caractéristiques décrites (même si cette sélection est isolée au sein d'une phrase comprenant ces autres caractéristiques), si cette sélection de caractéristiques est suffisante pour conférer un avantage technique ou pour différencier l'invention par rapport à l'état de la technique antérieure. Cette sélection comprend au moins une caractéristique, de préférence fonctionnelle sans détails structurels, ou avec seulement une partie des détails structurels si cette partie uniquement est suffisante pour conférer un avantage technique ou pour différencier l'invention par rapport à l'état de la technique antérieure.

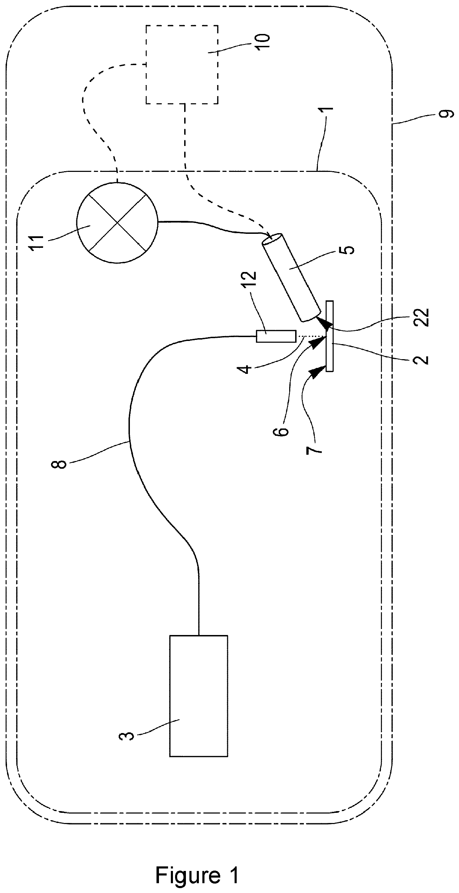

En référence à la FIGURE 1, il est décrit un dispositif pour la préparation d'un échantillon gazeux 1 obtenu à partir d'un échantillon solide 2. Le dispositif 1 comprend une source laser 3 agencée pour émettre un faisceau laser 4 apte à générer une calcination et/ou une combustion d'une partie de l'échantillon solide 2. Le dispositif 1 comprend également un moyen agencé pour collecter un échantillon gazeux 5 d'un gaz émis lors de la calcination et/ou de la combustion de la partie de l'échantillon solide 2. Le faisceau laser 4 présente une irradiance inférieure à 5 MW/cm² au niveau d'une zone d'impact donnée 6 sur la surface 7 de l'échantillon 2. Le dispositif 1 comprend également une fibre optique 8 pour la propagation du faisceau laser 4 entre la source laser 3 et la zone d'impact donnée 6 sur la surface 7 de l'échantillon 2.

En référence à la FIGURE 1, il est également décrit un dispositif de collecte de gaz produits et d'analyse isotopique 9 comprenant le dispositif pour la préparation d'un échantillon gazeux 1 et un spectromètre optique 10 agencé pour réaliser des analyses isotopiques d'éléments contenus dans l'échantillon gazeux préparé par le dispositif pour la préparation d'échantillons gazeux 1.

L'échantillon gazeux préparé selon l'invention est utilisé directement, sans aucune préparation, pour l'analyse isotopique. L'échantillon gazeux préparé selon l'invention n'est pas soumis à une étape de chromatographie en phase gazeuse préalablement à l'analyse isotopique.

Une diode laser fibrée 3 constitue la source laser 3 et la fibre optique 8 du dispositif pour la préparation d'un échantillon gazeux 1. La diode laser fibrée 3 est associée à un système de refroidissement et un contrôleur de courant. L'utilisation de fibre optique 8 n'est pas possible avec les sources lasers utilisées dans l'état de l'art, en particulier parce que les faisceaux lasers (leurs niveaux de puissance élevés) émis par les lasers solides endommagent les fibres optiques 8 et parce que les fibres optiques 8 en silice ne permettent pas la propagation des faisceaux lasers (dont la longueur d'onde est supérieure à 2 µm) émis par les lasers à gaz.

Trois diodes laser fibrées 3 ont été sélectionnées pour les essais. Les diodes laser fibrées 3 utilisées présentent des puissances respectives de 30 W pour les deux premières de 60 W pour la troisième. Les faisceaux laser 4 émis par les diodes laser fibrées 3 présentent des longueurs d'ondes respectives de 808 pour la première et 880 nm pour deux autres. Les diamètres respectifs de coeur des fibres des diodes laser fibrées 3 sont respectivement de 400 et 200 et 400 µm. L'ouverture numérique des fibres des diodes laser fibrées 3 est de 0,22. Le facteur M² du faisceau laser 4 calculé est ainsi de 80 pour une diode laser fibrée 3 émettant à 880 nm et ayant un diamètre de coeur de 200 µm de coeur. Le faisceau laser 4 est émis en mode continu. L'irradiance des faisceaux laser 4 des diodes laser fibrées 3 au niveau de la zone d'impact donnée 6 sur la surface 7 de l'échantillon 2 avec un télescope de grandissement 2 et une lentille de focalisation de 100 mm de distance focale est d'environ 10 à 20 kW/cm². Les faisceaux lasers 4 interagissant sur une profondeur maximale de l'échantillon solide 2 de 3 mm, les doses respectives reçues par l'échantillon 2 pendant une seconde est d'environ 50 kJ/cm³ et 100 kJ/cm³. Dans la suite de la description, la diode laser fibrée 3 présentant une puissance de 30 W à 880nm et 200 µm de diamètre de coeur a été utilisée.

L'irradiance de la diode laser fibrée 3 est suffisante pour chauffer la zone d'impact donnée 6 à une température proche de 900 °C de sorte à initier la réaction de calcination mais est insuffisante pour induire le phénomène d'ablation laser. Les lasers d'ablation étant fortement énergétiques, ils génèrent des espèces réactives qui se recombinent et/ou réagissent avec les isotopes d'intérêts à analyser. Les diodes laser fibrées 3 utilisées dans les conditions selon l'invention ont pour avantage d'éviter le fractionnement isotopique et donc la perte d'informations.

La taille du faisceau laser 4 au niveau de la zone d'impact donnée 6 sur la surface 7 de l'échantillon 2 est de l'ordre de quelques centaines de micromètres. Cela permet de réaliser une analyse localisée de l'échantillon solide 2.

L'échantillon solide 2 est une roche 2 contenant des carbonates. Contrairement aux méthodes de l'état de l'art, les roches ne sont pas traitées, ne sont pas préparées et ne sont soumises à aucun traitement de surface préalablement à la préparation de l'échantillon gazeux.

L'irradiation de la roche 2 par le faisceau laser 4 va provoquer la calcination de la roche 2 qui va dégager majoritairement du dioxyde de carbone qui sera collecté sous forme d'échantillon gazeux. Contrairement à la combustion, la réaction de calcination est une réaction qui, après avoir été initiée, est auto-entretenue et ne nécessite pas la présence d'oxygène.

L'analyse isotopique du carbone et/ou de l'oxygène sera représentative du type de roche analysée. Un autre avantage est la préparation quasi-uniquement sur la réaction de calcination, à travers l'utilisation de diode laser fibrée 3 dans les conditions selon l'invention, est d'obtenir une efficacité de réaction qui est de 100 % et de récupérer le maximum de dioxyde de carbone sans perdre d'informations. Les lasers d'ablation de l'état de l'art n'ont pas un rendement isotopique de 100 %.

Les lasers d'ablation induisent également des effets de matrice qui rendent la préparation de l'échantillon gazeux dépendante du type de roche 2. Un autre avantage lié au fait que la préparation de l'échantillon gazeux soit basée quasi-uniquement sur la réaction de calcination, est que la préparation n'est pas matrice dépendante.

Le dispositif pour la préparation d'échantillons gazeux 1 comprend des moyens de mise en forme et de focalisation 12 du faisceau laser 4 agencés pour que la distance focale du dispositif pour la préparation d'un échantillon gazeux 1 soit de 100 mm. Les moyens de mise en forme et de focalisation 12 comprennent, entre autres, des achromats. Le dispositif pour la préparation d'échantillons gazeux 1 comprend une pompe à vide 11, par exemple une pompe 11 à vide primaire à membrane ou à palettes qui est reliée au moyen de collecte d'échantillons gazeux 5 ou au spectromètre optique 10 lui-même relié au moyen de collecte d'échantillons gazeux 5. La pompe 11 permet de créer une dépression en entrée 22 du moyen de collecte d'un échantillon gazeux 5 permettant d'aspirer un gaz émis lors de la calcination et/ou de la combustion de la roche 2. Les moyens de mise en forme et de focalisation 12 du faisceau laser 4 sont également agencés pour que le dispositif pour la préparation d'échantillons gazeux 1 présente une longueur de Rayleigh égale à environ 2 mm.

La longueur de Rayleigh du dispositif pour la préparation d'échantillons gazeux 1 est telle qu'une variation de la distance entre la zone d'impact donnée 6 sur la surface 7 de la roche 2 et la sortie 18 des moyens de mise en forme et de focalisation 12 du faisceau laser 4 inférieure à 2 mm engendre une variation d'un waist du faisceau laser inférieur à 10 %.

Le rendement optique/électrique des diodes laser fibrées 3 est de l'ordre de 40 % ce qui permet d'obtenir des dispositifs pour la préparation d'échantillons gazeux 1 alimentés par batterie.

Les diodes laser fibrées 3 ont un prix qui est très nettement inférieur à celui des lasers solides ou à gaz.

Le fonctionnement des diodes laser fibrées 3 à basse tension et fort courant, typiquement 20 V maximum et 50 A maximum, permet d'utiliser une électronique associée aux diodes laser fibrées 3 compacte. L'encombrement réduit (de l'ordre de 3 dm³) de l'ensemble comprenant la diode laser fibrée 3, le dispositif Peltier et les contrôleurs associés permet de transporter le dispositif pour la préparation d'échantillons gazeux 1 ou le dispositif d'analyse isotopique 9 sur site. Cela permet de préparer des échantillons gazeux prêts à être analysés sur site ou à procéder à des analyses isotopiques directement sur site. L'utilisation d'une fibre optique 8 permet d'analyser des roches sur site dans des zones exiguës et/ou difficiles d'accès et/ou dans des zones non adaptées à de l'équipement électronique telles que, à titre d'exemple non limitatif, au niveau d'une tête de forage, dans des réseaux souterrains, dans des milieux avec de l'eau.

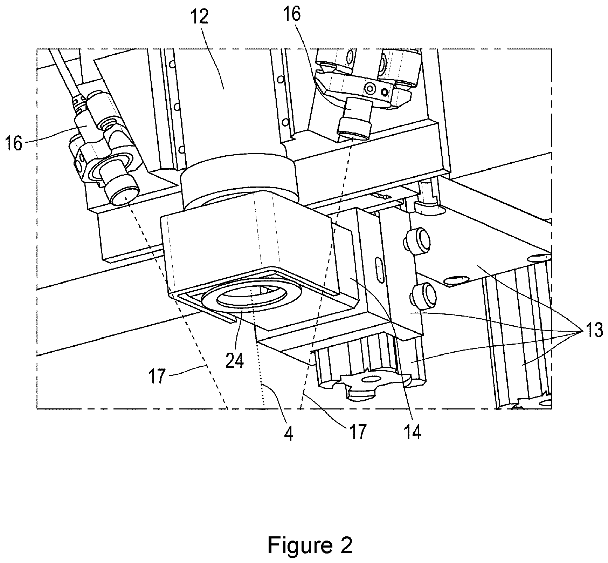

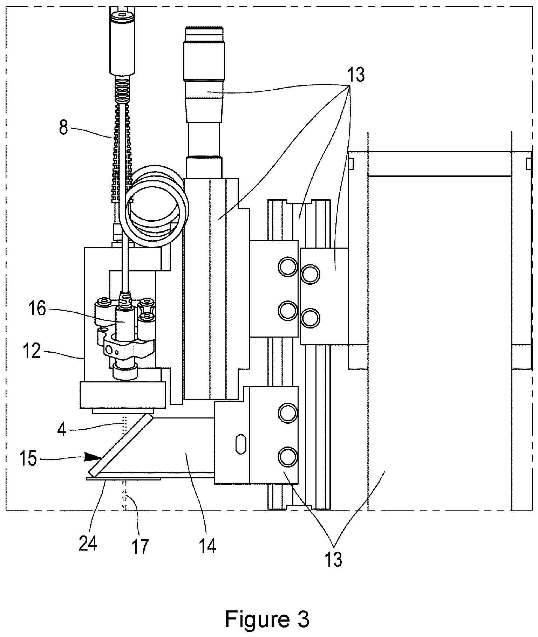

En référence aux FIGURES 2 et 3, le dispositif pour la préparation d'échantillons gazeux 1 comprend une table de guidage 13 agencée pour mettre en mouvement le faisceau laser 4 et/ou la zone d'impact 6 l'un relativement à l'autre.

Le dispositif pour la préparation d'échantillons gazeux 1 comprend une source de lumière à LED blanche 24 agencée pour illuminer au moins la zone d'impact 6. La source de lumière 24 est agencée sous forme d'anneau pour produire une illumination optimale de la surface 7 de la roche 2. Le dispositif pour la préparation d'échantillons gazeux 1 comprend également une caméra 14 agencée pour imager la zone d'impact illuminée. Le dispositif pour la préparation d'échantillons gazeux 1 comprend un miroir semi-réfléchissant 15 agencé pour réfléchir une partie de la lumière réfléchie par la surface 7 de la roche 2 vers la caméra 14.

Le dispositif pour la préparation d'échantillons gazeux 1 comprend également deux diodes lasers rouges 16 émettant des faisceaux lasers de visée 17 se croisant en un point unique dans le plan focal du dispositif pour la préparation d'échantillons gazeux 1.

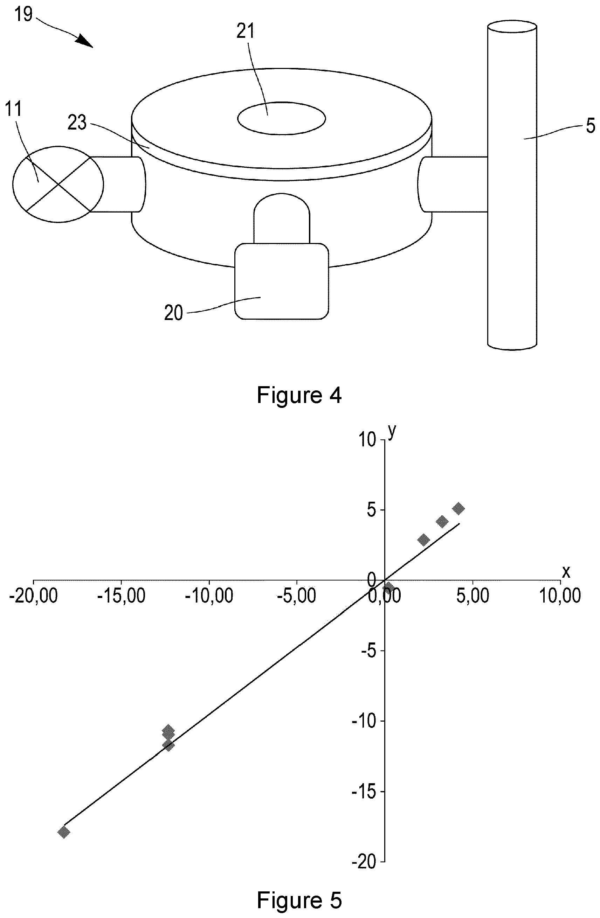

En référence à la FIGURE 4, il est proposé une enceinte 19 agencée pour que la roche 2 y soit disposée à l'intérieur. La roche 2 est disposée dans l'enceinte 19 par une ouverture fermée par un couvercle 23. L'enceinte 19 est agencée pour coopérer avec le moyen agencé pour collecter l'échantillon gazeux 5. Une première ouverture de l'enceinte 19 est connectée à la pompe 11 de sorte à pouvoir créer un vide primaire dans l'enceinte 19. Une deuxième ouverture de l'enceinte 19 est connectée à une jauge à vide 20. Le moyen agencé pour collecter l'échantillon gazeux 5 est connecté à une troisième ouverture de l'enceinte 19. La face supérieure de l'enceinte 19 comprend une fenêtre 21 en verre à travers laquelle se propagent, en direction de la roche 2 et/ou depuis la roche 2, la lumière émise par la LED blanche 24, la lumière réfléchie par la surface 6 de la roche 2, le faisceau laser 4 et les faisceaux lasers de visée 17.

Les tableaux 1 et 2 illustrent respectivement les résultats d'analyses isotopiques des ratios ¹³C/¹²C et ¹⁸O/¹⁶O mesurés par spectrométrie de masse sur des échantillons gazeux obtenus à partir des roches listées dans les tableaux 1 et 2. Ces rapports sont mesurés par rapport à un standard international International Atomic Energy Agency (IAEA) (le Pee Dee Belemnite (PDB)) et sont donnés sous forme de notation δ en pour mille. Dans les tableaux 1 et 2 sont présentés les résultats obtenus par un appareil commercial vendu sous la dénomination commerciale « KIEL IV Carbonate Device » de la marque « Thermo Scientific » comprenant une unité pour la préparation d'échantillons gazeux par traitement chimique selon l'état de la technique qui est couplé à un spectromètre de masse pour l'analyse du dioxyde de carbone préparé. Dans les tableaux 1 et 2 sont également présentés les résultats obtenus par le dispositif pour la préparation d'échantillons gazeux 1 selon l'invention couplé à l'enceinte 19 ; les échantillons gazeux ainsi préparés ont été analysés par un spectromètre de masse commercial Delta V Plus de chez « Thermo Scientific ». Il est également reporté dans les tableaux 1 et 2, la reproductibilité de la préparation selon l'invention lorsque la zone d'impact 6 varie sur le long de la surface 7 d'une même roche 2.

**[Table 1]**

| Rapport isotopique δ¹³C/¹²C en pour mille vs VPDB | | | |
|---|---|---|---|
| Nom de la roche analysée | Echantillon gazeux préparé par « KIEL IV Carbonate Device » | Echantillon gazeux préparé par le dispositif selon l'invention | Reproductibilité des mesures (1σ) |
| Calcite 2 | -0,25 | -0,635 | +/- 0,1 |
| Dolomie 2 | 3,31 | 4,06 | +/- 0,1 |
| Siderite 1 | -12,22 | -10,96 | +/- 0,1 |
| Malachite | -18,24 | -17,84 | +/- 0,1 |
| Siderite 2 | -12,27 | -11,73 | +/- 0,1 |
| Dolomite 1 | -12,22 | -10,66 | +/- 0,1 |
| Calcite 1 | 2,29 | 2,76 | +/- 0,1 |

**[Table 2]**

| Rapport isotopique δ¹⁸O/¹⁶O en pour mille vs VPDB | | | |
|---|---|---|---|
| Roche analysée | Echantillon gazeux préparé par « KIEL IV Carbonate Device » | Echantillon gazeux préparé par le dispositif selon l'invention | Reproductibilité des mesures (1σ) |
| Calcite 2 | -13,83 | -22,49 | +/- 0,2 |
| Dolomie 2 | -1,72 | -12,83 | +/- 0,2 |
| Siderite 1 | -14,57 | -22,03 | +/- 0,2 |
| Malachite | -3,3 | -14,02 | +/- 0,2 |
| Siderite 2 | -14,57 | -22,32 | +/- 0,2 |
| Dolomite 1 | -6,02 | -15,21 | +/- 0,2 |
| Calcite 1 | -3,38 | -13,38 | +/- 0,2 |

Il est à noter que les variations entre les mesures réalisées à différentes zones d'impact 6 sont substantiellement faibles. Cela démontre une excellente reproductibilité de la préparation d'échantillons gazeux selon l'invention.

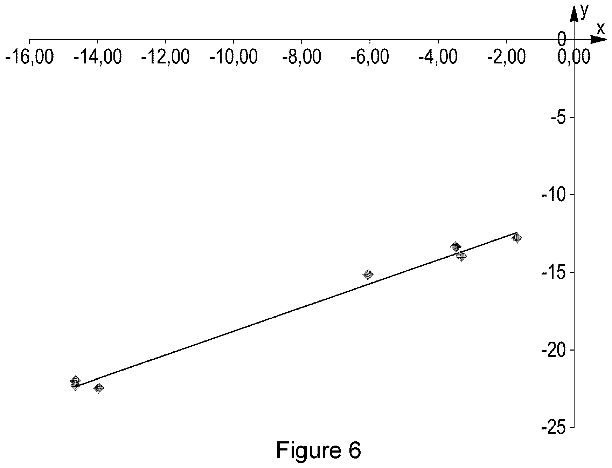

Les FIGURES 5 et 6 illustrent respectivement l'ajustement réalisé entre les ratios ¹³C/¹²C et ¹⁸O/¹⁶O mesurés par l'appareil commercial « KIEL IV Carbonate Device » sur les différentes roches du panel et les ratios ¹³C/¹²C et ¹⁸O/¹⁶O mesurés sur les échantillons gazeux préparés par le dispositif pour la préparation d'échantillons gazeux 1 selon l'invention à partir des différentes roches du panel. Les valeurs des ratios isotopiques mesurés sont renseignées dans les tableaux 1 et 2. L'axe des abscisses, noté x, illustre les ratios ¹³C/¹²C et ¹⁸O/¹⁶O mesurés par l'appareil commercial « KIEL IV Carbonate Device » et l'axe des ordonnées, noté y, illustre les ratios ¹³C/¹²C et ¹⁸O/¹⁶O mesurés sur les échantillons gazeux préparés par le dispositif pour la préparation d'échantillons gazeux 1 selon l'invention. Chaque point illustre les valeurs des ratios ¹³C/¹²C et ¹⁸O/¹⁶O mesurés sur un échantillon particulier tels que listés dans les tableaux 1 et 2. La droite en trait plein correspond à l'ajustement réalisé par régression linéaire entre les différents points.

Le coefficient de corrélation entre le ratio ¹³C/¹²C mesuré par l'appareil commercial « KIEL IV Carbonate Device » et le ratio ¹³C/¹²C mesuré sur l'échantillon gazeux préparé par le dispositif pour la préparation d'échantillons gazeux 1 est de 0,994. Le coefficient de corrélation entre le ratio ¹⁸O/¹⁶O mesuré par l'appareil commercial « KIEL IV Carbonate Device » et le ratio ¹⁸O/¹⁶O mesuré sur l'échantillon gazeux préparé par le dispositif pour la préparation d'échantillons gazeux 1 est de 0,989.

Les très bons coefficients de corrélation observés démontrent que la préparation des échantillons gazeux selon l'invention est équivalente à celle réalisée par l'appareil commercial « KIEL IV Carbonate Device » qui est considéré comme étant, à l'heure actuelle, un des appareils de référence pour l'analyse isotopique des roches carbonatées.

Bien sûr, l'invention n'est pas limitée aux exemples qui viennent d'être décrits et de nombreux aménagements peuvent être apportés à ces exemples sans sortir du cadre de l'invention, qui est indiqué dans les revendications annexées.

Ainsi, dans des variantes combinables entre elles des modes de réalisation précédemment décrits :
- la source laser 3 est une fibre dopée à pompage optique 3, et/ou
- le faisceau laser 4 est émis en mode impulsionnel relaxé et/ou haché ; en particulier pour limiter la quantité de gaz produit lors de la calcination et/ou de la combustion, et/ou
- le système de refroidissement associé à la source laser 3 est :
   - un module Peltier, ou
   - un système de refroidissement conductif, et/ou
- le miroir semi-réfléchissant 15 est un filtre passe haut qui est transparent à 45° d'incidence pour les longueurs d'ondes infrarouges et qui est réfléchissant pour les longueurs d'ondes visibles qui sont captées par la caméra 14, et/ou
- le dispositif pour la préparation d'échantillons gazeux 1 comprend un filtre passe-bas (de type KG3 non représenté) collé sur un objectif (non représenté) de la caméra 14 permet d'éliminer le rayonnement infrarouge de la diode laser fibrée 3 rétrodiffusé et réfléchi par la roche 2 lors de la préparation de l'échantillon gazeux, et/ou
- le dispositif d'analyse isotopique 9 comprend un spectromètre de masse 10 agencé pour réaliser des analyses isotopiques d'éléments contenus dans un échantillon gazeux préparé par le dispositif pour la préparation d'échantillon gazeux 1, et/ou
- le dispositif pour la préparation d'échantillons gazeux 1 comprend un élément optique situé sur un trajet du faisceau laser 4 en sortie 18 des moyens de mise en forme et de focalisation 12 du faisceau laser 4, l'élément optique est agencé pour protéger les moyens de mise en forme et de focalisation 12 du faisceau laser 4, et/ou
- le dispositif pour la préparation d'échantillons gazeux 1 comprend un moyen agencé pour déposer de la matière sur la zone d'impact 6 de l'échantillon solide 2, la matière étant agencée pour amorcer la calcination et/ou la combustion de la partie de l'échantillon solide 2 contiguë à la matière déposée ; cela est particulièrement utile lorsque l'échantillon solide 2 présente un faible coefficient d'absorption vis-à-vis du faisceau laser 4, et/ou
- le capteur optique 14 du dispositif pour la préparation d'échantillons gazeux 1 est agencé pour mesurer un ou des paramètres optiques de la lumière réfléchie par la surface 7 de l'échantillon solide 2 illuminée par la source laser 3 ou par une source lumineuse secondaire ou par la source à LED blanche 24, et/ou
- le dispositif pour la préparation d'échantillons gazeux 1 comprend une unité de traitement agencée et/ou configurée et/ou programmée pour déterminer, à partir du ou des paramètres optiques mesurés par le capteur optique 14, un flux lumineux à appliquer à la zone d'impact 6, et/ou
- la matière déposée sur la zone d'impact de l'échantillon solide 2 comprend des oxydes de fer, et/ou
- la première ouverture de l'enceinte est connectée à une source de gaz inerte 11, à titre d'exemple non limitatif d'azote, de sorte à créer une atmosphère inerte dans l'enceinte 19 en balayant l'enceinte 19 par un flux d'azote, et/ou
- un flux de gaz inerte peut être injecté, au moyen d'une arrivée de gaz, au niveau de la zone d'impact 6 de l'échantillon solide 2 de sorte à balayer la zone par un flux de gaz inerte durant la préparation ; cela a pour effet de rendre la préparation d'échantillon gazeux insensible à la présence de carbone organique dans ou à la surface 7 de l'échantillon solide 2, et/ou
- l'entrée 22 du moyen agencé pour collecter l'échantillon gazeux 5 est mise en contact avec la surface 7 de la roche 2 de sorte à fermer moyen agencé pour collecter l'échantillon gazeux 5 ; cela a pour effet de rendre la préparation d'échantillon gazeux insensible à la présence de carbone organique dans ou à la surface 7 de l'échantillon solide 2, et/ou
- un pourtour de l'entrée 22 du moyen agencé pour collecter l'échantillon gazeux 5 comprend un joint agencé pour être porté en appui direct contre la surface 7 de la roche 2 de sorte à fermer l'enceinte 19 hermétiquement.

De plus, les différentes caractéristiques, formes, variantes et modes de réalisation de l'invention peuvent être associés les uns avec les autres selon diverses combinaisons dans la mesure où ils ne sont pas incompatibles ou exclusifs les uns des autres.

## Revendications

1. Dispositif pour la préparation d'un échantillon gazeux (1) obtenu à partir d'un échantillon solide (2), ledit dispositif comprenant :
- une source laser (3) agencée pour émettre un faisceau laser (4) apte à générer une calcination et/ou une combustion d'une partie de l'échantillon solide et inapte à engendrer une ablation laser,
- un moyen agencé pour collecter un échantillon gazeux (5) d'un gaz émis lors de la calcination et/ou de la combustion de la partie de l'échantillon solide ; le dispositif étant **caractérisé en ce que** le faisceau laser présente une irradiance inférieure à 5 MW/cm² au niveau d'une zone d'impact donnée (6) sur une surface de l'échantillon (7), et **en ce qu'**il comprend une fibre optique (8) pour la propagation du faisceau laser (4) entre la source laser et la zone d'impact donnée (6) sur la surface de l'échantillor (7).

2. Dispositif selon la revendication 1, dans lequel un rendement optique/électrique de la source laser est supérieur à 30 %.

3. Dispositif selon la revendication 1 ou 2, dans lequel la source laser comprend une diode laser.

4. Dispositif selon la revendication précédente, dans lequel :
- la source laser est une diode laser, ou
- la source laser est une fibre dopée à pompage optique.

5. Dispositif selon l'une quelconque des revendications précédentes, dans lequel la source laser est agencée pour émettre un faisceau laser de longueur d'onde supérieure à 0,3 µm et/ou inférieure à 5 µm.

6. Dispositif selon l'une quelconque des revendications précédentes, dans lequel un diamètre d'un coeur de la fibre optique présente une valeur supérieure à 2 µm et/ou inférieure à 800 µm.

7. Dispositif selon l'une quelconque des revendications précédentes, dans lequel une ouverture numérique de la fibre optique est supérieure à 0,1 et/ou inférieure à 0,5.

8. Dispositif selon l'une quelconque des revendications précédentes, comprenant des moyens de mise en forme et de focalisation du faisceau laser étant agencés de sorte que le dispositif présente une distance focale supérieure à 5 mm.

9. Dispositif selon la revendication précédente, dans lequel la source laser, la fibre optique et les moyens de mise en forme et de focalisation sont agencés de sorte que le faisceau laser présente une longueur de Rayleigh supérieure à 0,5 mm et/ou inférieure à 10 mm.

10. Dispositif selon l'une quelconque des revendications précédentes, comprenant un moyen de mise en mouvement agencé pour mettre en mouvement le faisceau laser et/ou la zone d'impact l'un relativement à l'autre.

11. Dispositif selon l'une quelconque des revendications précédentes, dans lequel le faisceau optique présente un facteur M² supérieur à 2 et/ou inférieur à 200.

12. Dispositif selon l'une quelconque des revendications précédentes, comprenant :
- une source de lumière agencée pour illuminer au moins la zone d'impact,
- un capteur optique agencé pour imager la zone d'impact illuminée et/ou pour mesurer un ou des paramètres optiques d'une lumière réfléchie par la zone d'impact illuminée de l'échantillon solide.

13. Dispositif selon la revendication précédente, comprenant une unité de traitement agencée et/ou configurée et/ou programmée pour déterminer, à partir du ou des paramètres optiques mesurés par le capteur optique, un flux lumineux à appliquer à la zone d'impact.

14. Dispositif selon l'une quelconque des revendications précédentes, comprenant un moyen agencé pour déposer de la matière sur la zone d'impact de l'échantillon solide, ladite matière étant agencée pour amorcer la calcination et/ou la combustion de la partie de l'échantillon solide contiguë à la matière déposée.

15. Dispositif selon l'une quelconque des revendications précédentes, comprenant une enceinte agencée pour :
- coopérer avec le moyen agencé pour collecter l'échantillon gazeux,
- que l'échantillon solide y soit disposé à l'intérieur, ou
- être mise en contact direct avec la surface de l'échantillon solide.

16. Dispositif selon l'une quelconque des revendications précédentes, comprenant un élément optique situé sur un trajet du faisceau laser en sortie des moyens de mise en forme et de focalisation du faisceau laser, ledit élément optique étant agencé pour protéger lesdits moyens de mise en forme et de focalisation du faisceau laser.

17. Dispositif portable pour l'analyses de compositions isotopiques en carbone et/ou en oxygène contenus dans des roches carbonatées, ledit dispositif pour l'analyse de compositions isotopiques comprenant :
- le dispositif pour la préparation d'échantillon gazeux selon l'une quelconque des revendications 1 à 16,
- un spectromètre optique ou un spectromètre de masse agencé pour réaliser des analyses isotopiques d'éléments contenus dans des échantillons gazeux préparés par ledit dispositif pour la préparation d'échantillon gazeux.

18. Utilisation du dispositif pour la préparation d'un échantillon gazeux selon l'une des revendications 1 à 14, pour l'analyse de compositions isotopiques en carbone et/ou en oxygène contenus dans des roches carbonatées.

19. Procédé de préparation d'un échantillon gazeux obtenu à partir d'un échantillon solide, ledit procédé comprenant :
- une émission, par une source laser, d'un faisceau laser apte à générer une calcination et/ou une combustion d'une partie de l'échantillon solide et inapte à engendrer une ablation laser,
- une collecte, par un moyen de collecte, d'un échantillon gazeux d'un gaz émis lors de la calcination et/ou de la combustion de la partie de l'échantillon solide ;
le procédé étant **caractérisé en ce qu'**une irradiance du faisceau laser émis est inférieure à 5 MW/cm² au niveau d'une zone d'impact donnée sur une surface de l'échantillon,
et **en ce qu'**il comprend une propagation, par une fibre optique, du faisceau laser entre la source laser et la zone d'impact donnée sur la surface de l'échantillon.

## Patentansprüche

1. Vorrichtung zur Herstellung einer gasförmigen Probe (1), die aus einer festen Probe (2) gewonnen wird, wobei die Vorrichtung umfasst:
- eine Laserquelle (3), die dazu eingerichtet ist, einen Laserstrahl (4) zu emittieren, der dazu geeignet ist, eine Kalzinierung und/oder eine Verbrennung eines Teils der festen Probe zu erzeugen, und der nicht dazu geeignet ist, eine Laserablation zu verursachen,
- ein Mittel, das dazu eingerichtet ist, eine gasförmige Probe (5) eines Gases zu sammeln, das bei der Kalzinierung und/oder der Verbrennung des Teils der festen Probe freigesetzt wird;
wobei die Vorrichtung **dadurch gekennzeichnet ist, dass** der Laserstrahl eine Bestrahlungsstärke von weniger als 5 MW/cm² an einem gegebenen Auftreffbereich (6) auf einer Oberfläche der Probe (7) besitzt,
und dass sie eine optische Faser (8) für die Fortleitung des Laserstrahls (4) zwischen der Laserquelle und dem gegebenen Auftreffbereich (6) auf der Oberfläche der Probe (7) umfasst.

2. Vorrichtung nach Anspruch 1, wobei ein optisch/elektrischer Wirkungsgrad der Laserquelle größer als 30 % ist.

3. Vorrichtung nach Anspruch 1 oder 2, wobei die Laserquelle eine Laserdiode umfasst.

4. Vorrichtung nach dem vorhergehenden Anspruch, wobei:
- die Laserquelle eine Laserdiode ist, oder
- die Laserquelle eine optisch gepumpte, dotierte Faser ist.

5. Vorrichtung nach einem der vorhergehenden Ansprüche, wobei die Laserquelle dazu eingerichtet ist, einen Laserstrahl mit einer Wellenlänge von mehr als 0,3 µm und/oder weniger als 5 µm zu emittieren.

6. Vorrichtung nach einem der vorhergehenden Ansprüche, wobei ein Durchmesser eines Kerns der optischen Faser einen Wert von größer als 2 µm und/oder kleiner als 800 µm besitzt.

7. Vorrichtung nach einem der vorhergehenden Ansprüche, wobei eine numerische Apertur der optischen Faser größer als 0,1 und/oder kleiner als 0,5 ist.

8. Vorrichtung nach einem der vorhergehenden Ansprüche, umfassend Mittel zur Formung und Fokussierung des Laserstrahls, die derart eingerichtet sind, dass die Vorrichtung eine Fokuslänge von mehr als 5 mm besitzt.

9. Vorrichtung nach dem vorhergehenden Anspruch, wobei die Laserquelle, die optische Faser und die Mittel zur Formung und Fokussierung derart eingerichtet sind, dass der Laserstrahl eine Rayleigh-Länge von mehr als 0,5 mm und/oder weniger als 10 mm besitzt.

10. Vorrichtung nach einem der vorhergehenden Ansprüche, umfassend ein Bewegungsmittel, das dazu eingerichtet ist, den Laserstrahl und/oder den Auftreffbereich relativ zueinander zu bewegen.

11. Vorrichtung nach einem der vorhergehenden Ansprüche, wobei der optische Strahl einen M²-Wert von mehr als 2 und/oder weniger als 200 besitzt.

12. Vorrichtung nach einem der vorhergehenden Ansprüche, umfassend:
- eine Lichtquelle, die dazu eingerichtet ist, zumindest den Auftreffbereich zu beleuchten,
- einen optischen Sensor, der dazu eingerichtet ist, den beleuchteten Auftreffbereich abzubilden und/oder einen oder mehrere optische Parameter von Licht zu messen, das von dem beleuchteten Auftreffbereich der festen Probe reflektiert wird.

13. Vorrichtung nach dem vorhergehenden Anspruch, umfassend eine Verarbeitungseinheit, die dazu eingerichtet und/oder konfiguriert und/oder programmiert ist, aus dem oder den vom optischen Sensor gemessenen optischen Parametern einen auf den Auftreffbereich anzuwendenden Lichtfluss zu bestimmen.

14. Vorrichtung nach einem der vorhergehenden Ansprüche, umfassend ein Mittel, das dazu eingerichtet ist, Material auf den Auftreffbereich der festen Probe aufzubringen, wobei das Material dazu ausgelegt ist, die Kalzinierung und/oder die Verbrennung des an das aufgebrachte Material angrenzenden Teils der festen Probe zu initiieren.

15. Vorrichtung nach einem der vorhergehenden Ansprüche, umfassend ein Behältnis, das dazu eingerichtet ist:
- mit dem zum Sammeln der gasförmigen Probe eingerichteten Mittel zusammenzuwirken,
- dass die feste Probe darin angeordnet werden kann, oder
- in direkten Kontakt mit der Oberfläche der festen Probe gebracht zu werden.

16. Vorrichtung nach einem der vorhergehenden Ansprüche, umfassend ein optisches Element, das sich in einem Strahlengang des aus den Mitteln zur Formung und Fokussierung des Laserstrahls austretenden Laserstrahls befindet, wobei das optische Element dazu eingerichtet ist, die Mittel zur Formung und Fokussierung des Laserstrahls zu schützen.

17. Tragbare Vorrichtung für Analysen von Isotopenzusammensetzungen von Kohlenstoff und/oder Sauerstoff, die in Karbonatgestein enthalten sind, wobei die Vorrichtung für die Analyse von Isotopenzusammensetzungen umfasst:
- die Vorrichtung zur Herstellung einer gasförmigen Probe nach einem der Ansprüche 1 bis 16,
- ein optisches Spektrometer oder ein Massenspektrometer, das dazu eingerichtet ist, Isotopenanalysen von Elementen durchzuführen, die in gasförmigen Proben enthalten sind, die mittels der Vorrichtung zur Herstellung einer gasförmigen Probe hergestellt wurden.

18. Verwendung der Vorrichtung zur Herstellung einer gasförmigen Probe nach einem der Ansprüche 1 bis 14 für die Analyse von Isotopenzusammensetzungen von Kohlenstoff und/oder Sauerstoff, die in Karbonatgestein enthalten sind.

19. Verfahren zur Herstellung einer gasförmigen Probe, die aus einer festen Probe gewonnen wird, wobei das Verfahren umfasst:
- Emission, mittels einer Laserquelle, eines Laserstrahls, der dazu geeignet ist, eine Kalzinierung und/oder eine Verbrennung eines Teils der festen Probe zu erzeugen, und nicht dazu geeignet ist, eine Laserablation zu verursachen,
- Sammlung, mittels eines Sammelmittels, einer gasförmigen Probe eines Gases, das bei der Kalzinierung und/oder der Verbrennung des Teils der festen Probe freigesetzt wird;
wobei das Verfahren **dadurch gekennzeichnet ist, dass** die Bestrahlungsstärke des emittierten Laserstrahls in einem gegebenen Auftreffbereich auf einer Oberfläche der Probe weniger als 5 MW/cm² beträgt,
und dass es eine Fortleitung des Laserstrahls, mittels einer optischen Faser, zwischen der Laserquelle und dem gegebenen Auftreffbereich auf der Oberfläche der Probe umfasst.

## Claims

1. Device for preparing a gaseous sample (1) obtained from a solid sample (2), said device comprising:
- a laser source (3) arranged to emit a laser beam (4) which is capable of generating calcination and/or combustion of a portion of the solid sample and which is not capable of causing a laser ablation,
- a means arranged to collect a gaseous sample (5) of a gas emitted during the calcination and/or combustion of the portion of the solid sample;
the device being **characterized in that** the laser beam has an irradiance less than 5 MW/cm² in a given impact zone (6) on a surface of the sample (7),
and **in that** it comprises an optical fiber (8) for propagating the laser beam (4) between the laser source and the given impact zone (6) on the surface of the sample (7).

2. Device according to claim 1, wherein an optical/electrical efficiency of the laser source is greater than 30%.

3. Device according to claim 1 or 2, wherein the laser source comprises a laser diode.

4. Device according to the preceding claim, wherein:
- the laser source is a laser diode, or
- the laser source is an optically pumped doped fibre.

5. Device according to any one of the preceding claims, wherein the laser source is arranged to emit a laser beam with a wavelength greater than 0.3 µm and/or less than 5 µm.

6. Device according to any one of the preceding claims, wherein a diameter of a core of the optical fibre has a value greater than 2 µm and/or less than 800 µm.

7. Device according to any one of the preceding claims, wherein a numerical aperture of the optical fibre is greater than 0.1 and/or less than 0.5.

8. Device according to any one of the preceding claims, comprising means for shaping and focusing the laser beam being arranged such that the device has a focal distance greater than 5 mm.

9. Device according to the preceding claim, wherein the laser source, the optical fibre and the shaping and focusing means are arranged such that the laser beam has a Rayleigh length greater than 0.5 mm and/or less than 10 mm.

10. Device according to any one of the preceding claims, comprising a movement means arranged to move the laser beam and/or the impact zone relative to one another.

11. Device according to any one of the preceding claims, wherein the optical beam has an M² factor greater than 2 and/or less than 200.

12. Device according to any one of the preceding claims, comprising:
- a light source arranged to illuminate at least the impact zone,
- an optical sensor arranged to image the illuminated impact zone and/or to measure one or more optical parameters of light reflected by the illuminated impact zone of the solid sample.

13. Device according to the preceding claim, comprising a processing unit arranged and/or configured and/or programmed to determine, from the optical parameter(s) measured by the optical sensor, a luminous flux to be applied to the impact zone.

14. Device according to any one of the preceding claims, comprising a means arranged to deposit material on the impact zone of the solid sample, said material being arranged to initiate the calcination and/or combustion of the portion of the solid sample adjacent to the deposited material.

15. Device according to any one of the preceding claims, comprising an enclosure arranged:
- to cooperate with the means arranged to collect the gaseous sample,
- such that the solid sample is arranged inside it, or
- to be brought into direct contact with the surface of the solid sample.

16. Device according to any one of the preceding claims, comprising an optical element situated on a path of the laser beam at the output of the means for shaping and focusing the laser beam, said optical element being arranged to protect said means for shaping and focusing the laser beam.

17. Portable device for the analysis of carbon and/or oxygen isotopic compositions contained in carbonate rocks, said device for the analysis of isotopic compositions comprising:
- the device for preparing a gaseous sample according to any one of claims 1 to 16,
- an optical spectrometer or a mass spectrometer arranged to carry out isotopic analyses of elements contained in gaseous samples prepared by said device for preparing a gaseous sample.

18. Use of the device for preparing a gaseous sample according to one of claims 1 to 14, for the analysis of carbon and/or oxygen isotopic compositions contained in carbonate rocks.

19. Method for preparing a gaseous sample obtained from a solid sample, said method comprising:
- emission, by a laser source, of a laser beam which is capable of generating calcination and/or combustion of a portion of the solid sample and which is not capable of causing a laser ablation,
- collection, by a collection means, of a gaseous sample of a gas emitted during the calcination and/or combustion of the portion of the solid sample;
the method being **characterized in that** an irradiance of the laser beam emitted is less than 5 MW/cm² in a given impact zone on a surface of the sample,
and **in that** it comprises propagation, by an optical fibre, of the laser beam between the laser source and the given impact zone on the surface of the sample.
